(19) Europäisches Patentamt
European Patent Office
Office européen des brevets

(11) **EP 3 957 394 A1**

(12) **EUROPEAN PATENT APPLICATION**
published in accordance with Art. 153(4) EPC

(43) Date of publication:
**23.02.2022 Bulletin 2022/08**

(21) Application number: **20790994.6**

(22) Date of filing: **26.03.2020**

(51) International Patent Classification (IPC):
**B01J 23/887** (2006.01)     **B01J 37/02** (2006.01)
**B01J 37/08** (2006.01)     **C07B 61/00** (2006.01)
**C07C 253/26** (2006.01)     **C07C 255/08** (2006.01)

(52) Cooperative Patent Classification (CPC):
**B01J 23/887; B01J 37/02; B01J 37/08;**
**C07B 61/00; C07C 253/26; C07C 255/08;**
**Y02P 20/52**

(86) International application number:
**PCT/JP2020/013739**

(87) International publication number:
**WO 2020/213362 (22.10.2020 Gazette 2020/43)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **16.04.2019 JP 2019077512**

(71) Applicant: **Asahi Kasei Kabushiki Kaisha**
**Tokyo 1000006 (JP)**

(72) Inventors:
• **IITSUKA, Chihiro**
**Tokyo 100-0006 (JP)**
• **FUKUZAWA, Akiyoshi**
**Tokyo 100-0006 (JP)**

(74) Representative: **dompatent von Kreisler Selting**
**Werner -**
**Partnerschaft von Patent- und Rechtsanwälten**
**mbB**
**Deichmannhaus am Dom**
**Bahnhofsvorplatz 1**
**50667 Köln (DE)**

(54) **CATALYST, METHOD FOR MANUFACTURING CATALYST, AND METHOD FOR MANUFACTURING ACRYLONITRILE**

(57)     A catalyst comprising Mo, Bi, and Fe, and satisfying, in an X-ray diffraction analysis, $0.10 < P/R < 0.18$ and $0.06 < Q/R < 0.30$ where P represents a peak intensity at $2\theta = 22.9 \pm 0.2°$, Q represents a peak intensity at $2\theta = 28.1 \pm 0.1°$, and R represents a peak intensity at $2\theta = 26.6 \pm 0.2°$.

Figure 1

**Description**

Technical Field

The present invention relates to a catalyst, a method for producing a catalyst, and a method for producing acrylonitrile.

Background Art

As a method for producing acrylonitrile, ammoxidation of propylene is known. Additionally, this ammoxidation enables to obtain hydrogen cyanide together with acrylonitrile. As ammoxidation catalysts, oxide catalysts containing molybdenum, bismuth, and iron and oxide catalysts containing antimony and iron are utilized, and various modifications have been made on the catalysts having these basic compositions for the purpose of enhancing the ammoxidation reaction efficiency.

For example, the catalyst that forms a specific peak condition in an X-ray diffraction analysis described in Patent Literature 1 is considered to show high acrylonitrile selectivity and is less likely to exhibit decreased activity and selectivity in an extended period of reaction. In the ammoxidation reaction of propylene, isobutene, or tertiary butanol, the catalyst described in Patent Literature 2 containing specific metals and having a specific peak condition in an X-ray diffraction analysis is proposed to be capable of decreasing the production reaction of $CO_2$ and CO produced as side reaction products and preventing the decrease in object selectivity.

Citation List

Patent Literature

Patent Literature 1
Japanese Patent Laid-Open No. 2016-120468
Patent Literature 2
Japanese Patent Laid-Open No. 2012-245484

Summary of Invention

Technical Problem

As described above, the production of acrylonitrile by the ammoxidation also produces hydrogen cyanide. In the acrylonitrile production by the ammoxidation, enhancing the productivity of hydrogen cyanide in addition to acrylonitrile has been a challenge.

The present invention has been made in light of the above problem. The increase in an acrylonitrile yield and the increase in a hydrogen cyanide yield are in a trade-off relationship, and it is difficult to increase both of them. Thus, the present invention has an object to prevent the decrease in an acrylonitrile yield while enhancing a hydrogen cyanide yield in the ammoxidation reaction for producing acrylonitrile and hydrogen cyanide.

Solution to Problem

The present inventors conducted extensive studies to solve the problem and consequently found that the above problem can be solved by using a catalyst containing at least specific metal species and having peak intensity ratios within specific ranges in an X-ray diffraction analysis for the ammoxidation reaction, whereby the present invention was accomplished.

That is, the present invention is as follows.

[1] A catalyst comprising Mo, Bi, and Fe, and satisfying, in an X-ray diffraction analysis, 0.10 < P/R < 0.18 and 0.06 < Q/R < 0.30 where P represents a peak intensity at $2\theta = 22.9 \pm 0.2°$, Q represents a peak intensity at $2\theta = 28.1 \pm 0.1°$, and R represents a peak intensity at $2\theta = 26.6 \pm 0.2°$.

[2] The catalyst according to [1], comprising a metal oxide represented by a formula (1):

$$Mo_{12}Bi_aFe_bX_cY_dZ_eO_f \qquad (1)$$

wherein X is one or more elements selected from the group consisting of nickel, cobalt, magnesium, calcium, zinc, strontium, barium, and tungsten,

Y is one or more elements selected from the group consisting of cerium, chromium, lanthanum, neodymium, yttrium, praseodymium, samarium, aluminum, boron, gallium, and indium,

Z is one or more elements selected from the group consisting of sodium, potassium, rubidium, and cesium,

a, b, c, d and e satisfy $0.1 \leq a \leq 2.0$, $0.1 \leq b \leq 4.0$, $0.1 \leq c \leq 10.0$, $0.1 \leq d \leq 3.0$, and $0.01 \leq e \leq 2.0$, respectively, and

f is number of oxygen atoms required to satisfy valence requirements of other elements present.

[3] The catalyst according to [1] or [2], further comprising a silica-containing carrier.

[4] A method for producing the catalyst comprising Mo, Bi, and Fe according to [1] to [3], comprising:

a step of providing calcined particles obtained by calcining dried particles containing Mo, Bi, and Fe,

a Mo removal treatment step of removing at least a part of Mo-containing components from the calcined particles,

a step of subjecting the calcined particles from which the Mo-containing components have been removed to an impregnation operation to obtain impregnated particles, and

a step of calcining the impregnated particles.

[5] The method for producing the catalyst according to [4], wherein the particles are impregnated with elements by the impregnation operation, and the elements include an element dissolved out into a solvent used for the Mo removal treatment.

[6] The method for producing the catalyst according to [4] or [5], comprising, after the step of subjecting to the impregnation operation, a step of calcining the obtained impregnated particles in the air.

[7] A method for producing acrylonitrile, comprising a step of reacting propylene, molecular oxygen, and ammonia in the presence of the catalyst according to any one of [1] to [3].

[8] The method for producing acrylonitrile according to [7], which is carried out in a fluidized bed reactor.

[9] The method for producing acrylonitrile according to [7] or [8], wherein a molar ratio of ammonia and air to propylene ranges from 1.0/(0.8 to 2.5)/(7.0 to 12.0) in terms of propylene/ammonia/air ratio.

[10] The method for producing acrylonitrile according to any one of [7] to [9], wherein the reaction is carried out within a temperature range from 300 to 550°C.

Advantageous Effects of Invention

According to the catalyst of the present invention, a hydrogen cyanide yield can be enhanced while the decrease in an acrylonitrile yield is prevented in a method for producing acrylonitrile and hydrogen cyanide including a step of ammoxidating propylene.

Brief Description of Drawing

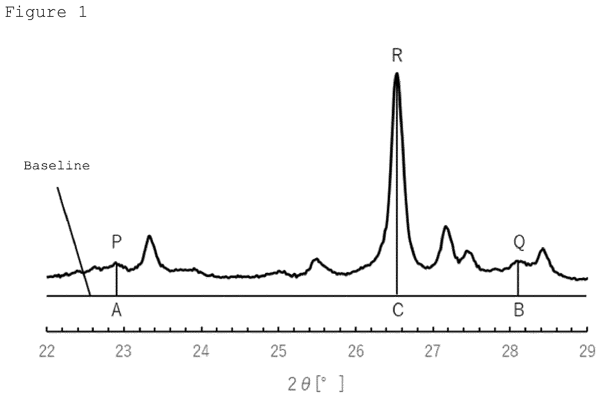

[Figure 1] Figure 1 shows a schematic view of a spectrum obtained by an X-ray diffraction analysis on the catalyst of the present invention.

Description of Embodiments

Hereinafter, embodiments of the present invention will be described in detail (hereinafter, referred to as the "present embodiment"). The present invention is not limited to the following present embodiments and can be carried out in various modifications within the range of subject matter. In the present description, the expression of "to" between numerical values or physical property values means to include those values before and after the "to". For example, a notation for a numerical value range represented as "1 to 100" includes both of "100" as the upper limit value and "1" as the lower limit value thereof. Similarly, the same applies to other notations for numerical value ranges.

[Catalyst]

The catalyst of the present embodiment is a catalyst containing Mo, Bi, and Fe and satisfying, in an X-ray diffraction analysis on the catalyst, $0.10 < P/R < 0.18$ and $0.06 < Q/R < 0.30$ where P represents a peak intensity at $2\theta = 22.9 \pm 0.2°$, Q represents a peak intensity at $2\theta = 28.1 \pm 0.1°$, and R represents a peak intensity at $2\theta = 26.6 \pm 0.2°$.

The catalyst of the present embodiment, when used in the method for producing acrylonitrile and hydrogen cyanide including a step of the ammoxidating propylene, can enhance a hydrogen cyanide yield while preventing the decrease in an acrylonitrile yield.

Hydrogen cyanide produced in the propylene ammoxidation is conceivably obtained from the decomposition of acrylonitrile as the main production route. When hydrogen cyanide is produced from acrylonitrile, acrylonitrile containing 3 carbon atoms produces 3 molecules of hydrogen cyanide containing 1 carbon atom, which is highly efficient carbon utilization, and this is important to efficiently produce hydrogen cyanide. In the present invention, the sum of a value of acrylonitrile yield and a value three times as much as a hydrogen cyanide yield can be an efficiency indicator of carbon utilization, whereby the compatibility between the prevention of decrease in an acrylonitrile yield and the enhancement in a hydrogen cyanide yield can be decided using this as the indicator.

The catalyst of the present embodiment shows characteristic peaks in an X-ray diffraction analysis. In the X-ray diffraction analysis of the present embodiment using CuKα line as the X-ray source at the anticathode, a spectrum as schematically shown in Figure 1 is obtained. At this time, the ordinate is diffraction X-ray intensity and the abscissa is 2θ value. The spectrum shown in Figure 1 is a schematic view to describe the peak intensities P, Q, and R in an X-ray diffraction analysis on the catalyst of the present embodiment. The spectrum shown in Figure 1 is not intended to limit the present invention.

The peak intensity P at 2θ = 22.9 ± 0.2° in the present embodiment is, as shown in Figure 1, the difference between the peak top point P at 2θ = 22.9 ± 0.2° on the diffraction pattern and a diffraction X-ray intensity at the intersection A when a perpendicular line is drawn to the baseline from the point P and is equivalent to the size of a line segment PA.

The peak intensity Q at 2θ = 28.1 ± 0.1° is, as shown in Figure 1, the difference between the peak top point Q at 2θ = 28.1 ± 0.1° on the diffraction pattern and a diffraction X-ray intensity at the intersection B when a perpendicular line is drawn to the baseline from the point Q and is equivalent to the size of a line segment QB.

Further, the peak intensity R at 2θ = 26.6 ± 0.2° is, as shown in Figure 1, the difference between the peak top point R at 2θ = 26.6 ± 0.2° on the diffraction pattern and a diffraction X-ray intensity at the intersection C when a perpendicular line is drawn to the baseline from the point R and is equivalent to the size of a line segment RC.

The peak top in the present description means the position at the maximum value in the spectrum within a predetermined 2θ range. When there are a plurality of peak tops within a predetermined 2θ range, the peak top in the present description is a peak top at the peak having the largest maximum value.

The baseline is a line, for example, connecting a peak-free point near 2θ = 10° to 15° and a peak-free point near 2θ = 35° to 40°.

The above "peak-free point" means a point on the so-called baseline on the chart with the ordinate being diffraction X-ray intensity and the abscissa being 2θ value in the XRD measurement and refers to the point at which a peak is absent. The typical XRD measurement includes noises and the like at a certain level, and thus a certain level of diffraction X-ray intensities are detected even at peak-free (diffraction spots are absent) areas. For this reason, a measuring apparatus is calculated, that is, noises and background are calculated from the entire analysis results to thereby mechanically determine the line at which diffraction X-ray intensities are substantially absent and define such a line as the baseline.

When the baseline is notably inclined against the X axis representing 2θ, the chart is suitably corrected using a supplied analysis software with the XRD instrument used for the measurement so that the baseline is parallel to the X axis to determine each of P, Q, and R peak intensities.

P/R in the present embodiment is the ratio of the peak intensity P at 2θ = 22.9 ± 0.2° to the peak intensity R at 2θ = 26.6 ± 0.2° and the value obtained by dividing the peak intensity P with the peak intensity R. P/R is more than 0.10, and preferably 0.11 or more. Additionally, the upper limit of P/R is less than 0.18, preferably 0.17 or less, and more preferably 0.15 or less. P/R more than 0.10 can enhance a hydrogen cyanide yield while preventing the decrease in an acrylonitrile yield.

Further, P/R can range from more than 0.10 and less than 0.18, more than 0.10 and 0.17 or less, more than 0.10 and 0.15 or less, and 0.11 or more and 0.15 or less.

Q/R in the present embodiment is the ratio of the peak intensity Q at 2θ = 28.1 ± 0.1° to the peak intensity R at 2θ = 26.6 ± 0.2° and the value obtained by dividing the peak intensity Q with the peak intensity R. Q/R is more than 0.06, preferably 0.07 or more, and more preferably 0.08 or more. The upper limit of Q/R is less than 0.30, preferably 0.25 or less, and more preferably 0.20 or less.

Further, Q/R can range from more than 0.06 and less than 0.30, more than 0.06 and 0.25 or less, 0.07 or more and 0.25 or less, and 0.08 or more and 0.25 or less.

P/R and Q/R being in the above ranges can enhance a hydrogen cyanide yield while preventing the decrease in an acrylonitrile yield in the method for producing acrylonitrile and hydrogen cyanide including a step of the ammoxidating propylene.

Example of the method for controlling P/R to be more than 0.10 and less than 0.18, and Q/R to be more than 0.06 and less than 0.30, include a method for producing the catalyst to be described later.

The catalyst of the present embodiment is not particularly limited as long as it contains at least Mo, Bi, and Fe and can also contain other elements.

Examples of other elements include magnesium, alkali metals and the like.

For example, the catalyst containing magnesium can stabilize the crystal phase and tends to decrease the transformation of the crystal phase to the $\alpha$ type, which leads to performance decline when subjected to the fluidized bed reaction. The catalyst containing an alkali metal tends to decrease the production of byproducts and keep a calcination temperature of the catalyst within a preferable zone.

The catalyst of the present embodiment preferably contains a metal oxide represented by the formula (1).

$$Mo_{12}Bi_aFe_bX_cY_dZ_eO_f \qquad (1)$$

wherein X is one or more elements selected from the group consisting of nickel, cobalt, magnesium, calcium, zinc, strontium, barium, and tungsten.

Y is one or more elements selected from the group consisting of cerium, chromium, lanthanum, neodymium, yttrium, praseodymium, samarium, aluminum, boron, gallium, and indium.

Z is one or more elements selected from the group consisting of sodium, potassium, rubidium, and cesium.

The element X serves to form molybdate that has suitable lattice defects and facilitate the oxygen movement in the bulk. The element Y can serve to provide the redox function in the catalyst as does iron. The element Z can serve to prevent the decomposition reaction of the main products and the starting materials by blocking the acid centers present on the catalyst surface.

Further, a, b, c, d and e satisfy $0.1 \leq a \leq 2.0$, $0.1 \leq b \leq 4.0$, $0.1 \leq c \leq 10.0$, $0.1 \leq d \leq 3.0$, and $0.01 \leq e \leq 2.0$, respectively.

f is the number of oxygen atoms required to satisfy valence requirements of other elements present.

The catalyst of the present embodiment can further contain elements other than the above.

The atomic ratio a of bismuth to 12 atoms of molybdenum is $0.1 \leq a \leq 2.0$, preferably $0.1 \leq a \leq 0.7$, and more preferably $0.15 \leq a \leq 0.7$.

When a is 0.1 or more and 2.0 or less, yields of acrylonitrile and hydrogen cyanide are higher and the reaction stability tends to be excellent.

The atomic ratio b of iron to 12 atoms of molybdenum is $0.1 \leq b \leq 4.0$, preferably $0.5 \leq b \leq 3.5$, and more preferably $1.0 \leq b \leq 3.5$.

The atomic ratio c of the element X to 12 atoms of molybdenum is $0.1 \leq c \leq 10.0$, preferably $3.0 \leq c \leq 9.0$, and more preferably $4.0 \leq c \leq 8.5$.

The atomic ratio d of the element Y to 12 atoms of molybdenum is $0.1 \leq d \leq 3.0$, preferably $0.2 \leq d \leq 2.0$, and more preferably $0.3 \leq d \leq 1.5$.

The atomic ratio e of the element Z to 12 atoms of molybdenum is $0.01 \leq e \leq 2.0$, and preferably $0.05 \leq e \leq 1.5$.

The atomic ratio f of oxygen to 12 atoms of molybdenum is the number of oxygen atoms required to satisfy valence requirements of other elements present.

The catalyst of the present embodiment can also be those in which a metal oxide is supported on a carrier. The carrier usable for the ammoxidation catalyst contains oxides such as silica, alumina, titania, and zirconia, but silica is preferable from viewpoints of low object selectivity decrease and good abrasion resistance and particle strength of the formed catalyst particles. That is, one of the preferable aspects of the catalyst of the present embodiment is the catalyst further containing silica.

The amount of the silica carrier used with respect to the total mass of the silica carrier and a metal oxide ranges typically from 20 mass% to 80 mass%, preferably 30 mass% to 70 mass%, and further preferably 40 mass% to 60 mass%.

The starting material of silica carrier is not particularly limited and examples include a silica sol (also called colloidal silica) and powder silica. The starting material of silica carrier is preferably a silica sol from a viewpoint of easy handleability. The average primary particle size of silica contained in a silica sol is not particularly limited. For the silica carrier, silica sols having different average primary particle sizes can be used in mixture.

The shape of the catalyst of the present embodiment is not particularly limited and, when used as a fluidized bed catalyst, preferably spherical from a viewpoint of fluidity. The particle size of the catalyst of the present embodiment is preferably 10 to 150 $\mu$m in terms of median diameter.

The median diameter of the catalyst of the present embodiment can be obtained by adding 0.6 g of the catalyst to 250 ml of water as dispersion medium, performing ultrasonic dispersion for 1 minute and then measuring using a laser diffraction/scattering particle size distribution analyzer LA-300, manufactured by HORIBA, Ltd., under a condition of a relative refractive index of 1.40.

[Method for producing the catalyst]

One of the present embodiments is a method for producing the catalyst comprising Mo, Bi, and Fe, the method including a step of providing calcined particles obtained by calcining dried particles containing Mo, Bi, and Fe (hereinafter, also referred to as Step I),

a step of removing at least a part of Mo-containing components from the calcined particles (hereinafter, also referred to as Step II),
a step of subjecting the calcined particles from which the Mo-containing components have been removed to an impregnation operation to obtain impregnated particles (hereinafter, also referred to as Step III), and
a step of calcining the impregnated particles (hereinafter, also referred to as Step IV). The production method of the present embodiment can produce the catalyst of the present embodiment described above.

(Step I)

Step I in the production method of the present embodiment is a step of providing calcined particles obtained by calcining dried particles containing Mo, Bi, and Fe. The calcined particles can be prepared by obtaining ready-made products or can be prepared by spray drying a slurry containing Mo, Bi, and Fe to obtained dried particles and calcining such dried particles.

The slurry containing Mo, Bi, and Fe can be obtained by mixing starting materials of the catalyst and a solvent. The solvent is preferably water, and the slurry is preferably an aqueous slurry. When the carrier is silica, a preparation method of mixing and stirring an aqueous solution containing molybdenum with an aqueous solution containing silica and then mixing and stirring a solution containing bismuth and other metals therewith is preferably used.

Further, additives can also be added when preparing a solution of each starting material and the slurry. Examples of the additive include organic aids.

The starting material of each element constituting the catalyst such as molybdenum, bismuth, and, iron, or optionally contained cerium, nickel, cobalt, magnesium, zinc, potassium, rubidium, calcium, and cesium for preparing the slurry is a salt soluble in water or nitric acid, and examples include an ammonium salt, a nitrate, a hydrochloride, a sulfate, and an organic acid salt of each metal.

An ammonium salt is preferably used as the starting material containing molybdenum, and a nitrate is preferably used as the starting material containing bismuth, cerium, iron, nickel, magnesium, zinc, potassium, rubidium, and cesium.

The slurry containing Mo, Bi, and Fe is spray dried to thereby prepare dried particles. In the spray drying, the slurry is spray dried whereby spherical particles are obtained. The spraying of aqueous slurry can be carried out by typically used industrial centrifugal method, two-fluid nozzle method, and high pressure nozzle method, and the spraying is preferably carried out by the centrifugal method. For drying, a heated air is preferably used, and examples of the heat source for drying include steam and an electric heater. The inlet temperature of a dryer is preferably 100°C to 400°C, and more preferably 150°C to 300°C. The outlet temperature of a dryer is preferably 100°C to 180°C, and more preferably 110°C to 170°C.

The dried particles obtained as described above are calcined. At this time, the dried particles are preferably calcined in the air. The calcination of dried particles in the air is carried out at temperature ranges preferably from 150°C to 750°C, and preferably carried out separately by precalcination and postcalcination without limiting particular conditions. In the precalcination, the calcination is preferably carried out under conditions of 150°C to 450°C for 30 minutes to 10 hours, and in the postcalcination, the calcination is preferably carried out under conditions of 500°C to 700°C, and preferably 520°C to 650°C for 1 hour to 20 hours. Air is used as the atmospheric gas at the calcination. A calcination furnace such as an electric furnace can be used for the calcination in the air.

(Step II)

Step II in the production method of the present embodiment is a step of removing at least a part of MO-containing components from the calcined particles obtained by Step I, and more specifically a step of removing or decreasing Mo-containing components present on the surface of calcined particles (hereinafter, referred to as "Mo removal treatment step".) The method of removing Mo-containing components is not particularly limited, and examples include a method of allowing the calcined particles obtained by Step I to contact a solvent or a solution and obtaining particles subjected to the Mo removal treatment or decreasing treatment. Undergoing Step II enables to effectively carry out Step III and Step IV to be described later and enhance a hydrogen cyanide yield. Examples of the Mo-containing component include molybdate compounds having $Bi_2Mo_3O_{12}$ and $Fe_2MO_3O_{12}$ as the main components.

The surface of calcined particles herein refers to the contactable part by gases such as propylene, ammonia, oxygen, and air and liquids such as solvents and solutions to be described later. Thus, when the calcined particle is a spherical shape, not only the spherical surface constituting the entire outer edge of the calcined particle but also the surface of pore structure formed in the calcined particle is included.

The removal or decrease of Mo-containing components present on the surface of calcined particles obtained by Step I by the Mo removal treatment step can form and grow well the $Bi_3FeMo_2O_{12}$ phase on the surface of treated calcined particles in Step IV (calcination step). More specifically, the removal or decrease of molybdate compounds having $Bi_2Mo_3O_{12}$ or $Fe_2MO_3O_{12}$ as the main component present on the surface of calcined particles obtained by Step I can form and grow well the $Bi_3FeMo_2O_{12}$ phase on the surface of treated calcined particles in Step IV (calcination step). That is, the catalyst satisfying $0.10 < P/R < 0.18$ and $0.06 < Q/R < 0.30$ as defined in the present invention can be finally obtained. Thus, the well formation and growth of $Bi_3FeMo_2O_{12}$ phase on the surface of treated calcined particles can enhance a hydrogen cyanide yield while preventing the decrease in an acrylonitrile yield in the ammoxidation reaction using the catalyst of the present invention.

For the well formation and growth of the $Bi_3FeMo_2O_{12}$ phase, Mo-containing components on the particle surface before the calcination step of Step IV need to be insufficient, and a technique used in the Mo removal treatment step is not limited to the aspects described below. Hereinafter, explanation will be made with a treatment of removing or decreasing Mo-containing components by allowing the calcined particles to contact a solvent or a solution as an example.

The solvent used in the Mo removal treatment step is not particularly limited and examples include water and alcohols. Water is preferably used as the solvent. When a solution is used, water is preferable as the solvent, a basic compound is preferable as the solute, with a basic inorganic compound being more preferable. Examples of the solute specifically include sodium hydroxide, potassium hydroxide, magnesium hydroxide, sodium carbonate, potassium carbonate, magnesium carbonate, calcium carbonate, barium carbonate, sodium hydrogen carbonate, potassium hydrogen carbonate, and ammonia. These solutes can be used singly, or in combinations of two or more thereof.

The solute content in the solution is not particularly limited and can be suitably adjusted depending on the solute used. The solute content in the solution is preferably 0.01 mass% or more and 10 mass% or less, more preferably 0.02 mass% or more and 1 mass% or less, and further preferably 0.02 mass% or more and 0.5 mass% or less. The pH of the solution is preferably 9 to 13, and more preferably 10 to 12. When a solute content is 0.01 mass% or more and 10 mass% or less, elements in the calcined particles suitably dissolve out into the solution and the impregnation in the subsequent step is sufficiently caused. For this reason, in the production of acrylonitrile and hydrogen cyanide by the propylene ammoxidation, the catalyst capable of enhancing a hydrogen cyanide yield while preventing the decrease in an acrylonitrile yield tends to be obtained.

The contact of the calcined particles and the solvent or the solution can be carried out by mixing the calcined particles and the solvent or the solution. The calcined particles can also be stirred in the solvent or the solution as needed. The calcined particle amount (g) to the solvent amount or the solution amount (mL) is not particularly limited and typically 0.01 g/mL or more and 10 g/mL or less, preferably 0.05 g/mL or more and 5 g/mL or less, and more preferably 0.05 g/mL or more and 1 g/mL or less. When a calcined particle amount to a solvent amount or a solution amount (mL) is 0.01 g/mL or more and 10 g/mL or less, elements in the calcined particles suitably dissolve out into the solvent or the solution and the impregnation in the subsequent step is sufficiently caused. For this reason, in the production of acrylonitrile and hydrogen cyanide by the propylene ammoxidation, the catalyst capable of enhancing a hydrogen cyanide yield while preventing the decrease in an acrylonitrile yield tends to be obtained.

The contact temperature of the calcined particles and the solvent or the solution is not particularly limited and is, from a viewpoint of accelerating the dissolution of elements in the calcined particles out into the solvent or the solution, preferably 10 to 100°C, more preferably 20 to 80°C, and further preferably 30 to 70°C. The contact time of the calcined particles and the solvent or the solution is not particularly limited and is, from a viewpoint of accelerating the dissolution of elements in the calcined particles out into the solvent or the solution, preferably 1 minute to 24 hours, more preferably 10 minutes to 10 hours, and further preferably 30 minutes to 2 hours.

After the contact with the solvent or the solution, the treated particles are separated by filtration from the solvent or the solution to thereby obtain as the Mo-removed particles. At this time, the Mo-removed particles can also be washed with water to remove the solvent or the solution. The Mo-removed particles can also be dried under atmospheric pressure or in a vacuum, preferably in a vacuum, at 10 to 200°C, preferably 50 to 100°C for 1 to 50 hours, preferably 5 to 30 hours. These operations carried out after the contact with the solvent or the solution can enhance the effect of impregnation treatment in the subsequent Step III.

(Step III)

Step III in the production method of the present embodiment is a step of subjecting the particles obtained by Step II to an impregnation operation to obtain impregnated particles. The "impregnation" herein refers to impregnation of the inner part of pores present in the solvent-treated particles with elements to be impregnated.

The impregnation treatment can be specifically carried out by mixing the particles obtained by Step II and an impregnation solution and vigorously shaking. The impregnation solution is a solution containing elements to be impregnated and preferably contains, as such elements, the elements dissolved out by Step II. Thus, the impregnation solution can preferably contain molybdenum, bismuth, and iron, and can optionally contain elements such as cerium, nickel, cobalt, magnesium, zinc, potassium, rubidium, calcium, and cesium. Of these, rubidium, molybdenum, and calcium are more preferable. The starting material of each element is a salt soluble in water or nitric acid, and an ammonium salt, a nitrate, a hydrochloride, a sulfate, and an organic acid salt of each metal can be used. The concentration of elements in the impregnation solution is not particularly limited and is, based on the number of Mo moles of 12 of the calcined particles obtained by Step I in which the impregnation treatment is carried out, typically 0.01 to 1.0, and preferably 0.05 to 0.6, in terms of the number of moles, with a salt of each metal as the element starting material being a substrate.

The amount of impregnation solution is preferably 0.5 times or more and 10 times or less, preferably 1.0 time or more and 5.0 times of less, and further preferably 1.3 times or more and 2.0 times or less, of a pore volume of the calcined particles before the solvent treatment. When an amount of the impregnation solution is 0.5 times or more and 10 times or less of the pore volume, the impregnation is sufficiently caused and the catalyst capable of enhancing a hydrogen cyanide yield while preventing the decrease in an acrylonitrile yield in the production of acrylonitrile and hydrogen cyanide by the propylene ammoxidation tends to be obtained.

Further, the pore volume of calcined particles is preferably 0.07 cc/g or more and 0.30 cc/g or less, and further preferably 0.10 cc/g or more and 0.20 cc/g or less.

The pore volume can be measured by the one-point method after 18-hour pretreatment using a Micro Meritics 3-Flex with 0.5 g of a catalyst amount and an $N_2$ gas as an adsorption gas under the conditions of 250°C and 0.001 mmHg or less.

The vigorous shaking for the impregnation can be carried out by, for example, stirring with vigorous shaking by hands, stirring using a vortex mixer, an ultrasonic wave or the like, or using other general impregnating equipment. The impregnation operation can be carried out regardless of under atmospheric pressure, a vacuum, or applied pressure. The duration of shaking is not particularly limited and typically 10 seconds to 30 minutes, and preferably 1 minute to 10 minutes.

The obtained impregnated particles can also be dried under atmospheric pressure or in a vacuum, preferably in a vacuum, at 10 to 200°C, preferably 30 to 100°C for 1 to 50 hours, preferably 5 to 30 hours. The drying enables the elements to settle on the solvent-treated particle surfaces and the inner part of pores.

(Step IV)

Step IV of the production method of the present embodiment is a step of calcining the impregnated particles obtained by Step III.

The impregnated particles are preferably calcined in the air. The calcination of impregnated particles is preferably carried out in a temperature range from 150°C to 750°C. The calcination is preferably carried out under the condition of calcination time of 10 minutes to 10 hours. The calcination in the air can use a calcination furnace such as an electric furnace.

(Other steps)

In the production method of the present embodiment, Mo can be added to and reacted with the catalyst after Step IV within the range of not affecting the actions and the effects of the present invention. The Mo added is not particularly limited, and complex oxides and catalysts containing a large amount of an ammonium salt, an oxide, and Mo can be used.

[Method of producing acrylonitrile and hydrogen cyanide]

The method for producing acrylonitrile of the present embodiment uses the catalyst of the present embodiment. That is, the method for producing acrylonitrile of the present embodiment includes a step of reacting propylene, molecular oxygen and ammonia in the presence of the catalyst of the present embodiment. The production method of the present embodiment is preferably carried out by the fluidized bed ammoxidation reaction. The method of producing acrylonitrile of the present embodiment can produce acrylonitrile and hydrogen cyanide.

The method for producing acrylonitrile of the present embodiment is preferably carried out, for example, in the typically used fluidized bed reactor. The starting materials, propylene and ammonia, do not necessarily need to be in high purity and those of industrial grades can be used. Further, it is typically preferable to use the air as the molecular oxygen source, but a gas having an increased oxygen concentration by mixing oxygen with the air can also be used.

When a molecular oxygen source is the air in the method for producing acrylonitrile according to the present embodiment, the composition of starting material gases (molar ratio of ammonia and air to propylene) ranges, in terms of propylene/ammonia/air ratio, preferably 1.0/(0.8 to 2.5)/(7.0 to 12.0), and more preferably 1.0/(0.8 to 1.4)/(8 to 11).

The reaction temperature in the method for producing acrylonitrile according to the present embodiment ranges preferably from 300 to 550°C, and more preferably from 400 to 500°C. The reaction pressure ranges preferably from atmospheric pressure to 0.3 MPa. The contact time of the starting material gases and the catalyst is preferably 0.5 to 20 (sec·g/cc), and more preferably 1 to 10 (sec·g/cc).

Examples

Hereinafter, the present embodiment is more specifically described in reference to the examples but is not at all limited to these examples. The evaluation methods for various physical properties are as described below.

[X-Ray diffraction analysis (XRD Analysis)]

XRD Analysis on the catalyst obtained in Examples and Comparative Examples was carried out under the following conditions. The catalysts were measured as they were without pulverization. When the catalyst was pulverized, the impact caused the $\beta$ type divalent metal molybdate crystal phase to transfer to the $\alpha$ type thereby failing to obtain the original diffraction pattern.

Measurement conditions

Detector: one-dimensional semiconductor detector LynxEye
Capture angle: 3°
Pixel size: 75 $\mu$m
Tube: Cu
Tube voltage: 40 kV
Tube current: 40 mA
Divergence slit: 0.3°
Step width: 0.02°/step (10° to 70° measurement)
Measurement time: 0.5 sec/step
Number of measured particles: about 250000 particles
Sample rotation: 15 rpm

[Reaction conditions and yield of acrylonitrile production by the propylene ammoxidation reaction]

Using the catalyst obtained in Examples and Comparative Examples, acrylonitrile and hydrogen cyanide were produced by the propylene ammoxidation reaction. For the reaction tube used during the reaction, a Pyrex (registered trademark) glass tube with 16 built-in 10-mesh wire meshes at 1 cm interval and having an inner diameter of 25 mm was used.

With a catalyst amount of 50 cc, a reaction temperature of 430°C, and a reaction pressure of 0.17 MPa being preset, a mixed gas of propylene/ammonia/air was supplied at 250 to 450 cc/sec (in terms of NTP) as the total gas flow rate to carry out the reaction. During this operation, the propylene content in the mixed gas was 9 vol% and the molar ratio of propylene/ammonia/air was 1/(0.7 to 1.4)/(8.0 to 13.5), and within which ranges, an ammonia flow rate was suitably changed so that a sulfuric acid consumption rate defined by the following formula was 20 $\pm$ 2kg/T-AN, and an air flow rate was suitably changed so that an oxygen concentration in the reactor outlet gas was 0.2 $\pm$ 0.02 vol%. At this time, the molar ratio of ammonia/propylene was defined as N/C. Further, the flow velocity of the entire mixed gas was changed to change the contact time defined by the following formula and set so that the propylene conversion rate defined by the following formula was 99.3 $\pm$ 0.2%.

The acrylonitrile yield produced by the reaction were the values defined as the following formulae.

$$\text{Sulfuric acid consumption rate (kg/T-AN)}$$
$$\frac{\text{Weight of sulfuric acid required to neutralize unreacted ammonia (kg)}}{\text{Production weight of acrylonitrile (T)}}$$

$$\text{Contact time (sec.)} = \frac{\text{Catalyst amount (cc)}}{\text{Mixed gas flow rate (cc-NTP/sec.)}} \times \frac{2\ 7\ 3}{273 + \text{reaction temperature (°C)}}$$

$$\times \frac{\text{Reaction pressure (MPa)}}{0.\ 1\ 0}$$

$$\text{Propylene conversion rate (\%)} = \frac{\text{Consumed propylene (mol)}}{\text{Supplied propylene (mol)}} \times 1\ 0\ 0$$

$$\text{Acrylonitrile yield (\%)} = \frac{\text{Produced acrylonitrile (mol)}}{\text{Supplied propylene (mol)}} \times 1\ 0\ 0$$

[Production Example 1]

A catalyst (calcined particle) in which a metal oxide having a composition represented by $Mo_{12}Bi_{0.39}Fe_{1.7}Ni_{4.2}Co_{3.7}Ce_{0.90}Rb_{0.14}O_f$ was supported on 40 mass% of silica was produced.

Specifically, ammonium heptamolybdate was dissolved in warm water (Solution A). Further, bismuth nitrate, iron nitrate, nickel nitrate, cobalt nitrate, cerium nitrate, and rubidium nitrate were dissolved in a nitric acid aqueous solution having 16.6 mass% (Solution B). For the silica to be a carrier, a silica sol in which silica having an average particle size of primary particles of 12 nm were dispersed was used. Solution A was added to the silica sol, and then Solution B was added thereto, stirred and mixed. Subsequently, using a sprayer equipped with a disc rotor installed at the center of the upper part of a dryer, the aqueous raw material mixture was spry dried under conditions of an inlet temperature of about 230°C and an outlet temperature of about 120°C. Then, the dried catalyst precursor was precalcined in an electric furnace under an air atmosphere at 320°C for 2 hours and subsequently finally calcined under an air atmosphere at 590°C for 2 hours.

The obtained calcined particle was a spherical shape and had an average particle size of 54 $\mu$m. The average particle size was measured using a laser diffraction/scattering particle size distribution analyzer LA-300, manufactured by HORIBA, Ltd.

Additionally, when acrylonitrile was produced by the propylene ammoxidation reaction using the obtained calcined particles as the catalyst, an acrylonitrile (AN) yield was 84.0% and a hydrogen cyanide (HCN) yield was 3.1%, and an AN + 3HCN (the sum of the value of AN yield and the tripled value of HCN yield) was 93.3.

[Examples 1 to 13 and Comparative Examples 1 to 9]

The calcined particle obtained by Production Example 1 was subjected to the following (Mo removal treatment operation) using the solvent shown in Table 1 to thereby obtain Mo-removal treated particles. Subsequently, using an aqueous solution containing an element to be impregnated shown in Table 1, the Mo-removal treated particles were subjected to the impregnation operation as described in the following (Impregnation operation) to thereby obtain impregnated particles. Further, calcination was performed under an air atmosphere at 580°C for 2 hours to thereby obtain a catalyst. The aqueous solution containing an element to be impregnated in each of Examples and Comparative Examples was prepared using, as the solute, rubidium nitrate when the impregnation element was Rb, potassium nitrate when the impregnation element was K, ammonium hepta molybdate when the impregnation element was Mo, calcium nitrate when the impregnation element was Ca. At this time, an amount of the solute in the impregnation solution was 0.06 to 0.4 based on the number of Mo moles of 12.

(Mo Removal treatment operation)

70 g of the calcined particles obtained by Production Example 1 and 560 mL of the solvent were stirred for 1 hour while retaining at 50°C. Subsequently, the solvent-treated catalyst was separated by filtration and washed with water and dried in a vacuum at 80°C for 15 hours.

(Impregnation operation)

A solution containing an element to be impregnated, that is, an impregnation solution, was adjusted in such a way as to be 1.6 times a solution amount of the pore volume of the catalyst to be impregnated (pre-solvent treatment). Subsequently, the impregnation solution was added to the solvent-treated catalyst and vigorously shaken for 5 minutes to contact and mix thoroughly the impregnation solution and the Mo-removal treated catalyst. Then, drying was carried out in a vacuum at 60°C for 2 hours.

As shown in Table 1, the above (Solvent treatment operation) was not carried out in Comparative Example 9 and the above (Impregnation operation) and the subsequent calcination were not carried out in Comparative Examples 1 to 8. The obtained catalysts were XRD analyzed by the method described earlier to thereby determine P/R and Q/R values. The P/R and Q/R values are shown in Table 1. Further, using the obtained catalysts, acrylonitrile was produced by the propylene ammoxidation reaction. Table 1 shows AN yields, HCN yields, AN + 3HCN values, and differences from the AN + 3HCN values of the calcined particles obtained in Production Example 1.

[Table 1]

| | Solvent treatment and impregnation condition | | X-Ray Diffraction | | Reaction evaluation result | | | |
|---|---|---|---|---|---|---|---|---|
| | Treatment solvent | Impregnation element | P/R | Q/R | AN Yield [%] | HCN Yield [%] | AN + 3HCN | AN + 3HCN Difference from Production Example 1 |
| Example 1 | Water | Rb | 0.12 | 0.08 | 82.1 | 4.3 | 95.0 | 1.7 |
| Example 2 | Water | K | 0.12 | 0.07 | 81.8 | 4.1 | 94.1 | 0.8 |
| Example 3 | 0.05%NaOH Aqueous solution | Rb | 0.11 | 0.08 | 81.1 | 4.6 | 94.9 | 1.6 |
| Example 4 | 0.05% NH$_3$ Aqueous solution | Rb | 0.11 | 0.09 | 80.2 | 4.9 | 94.9 | 1.6 |
| Example 5 | 0.1% NH$_3$ Aqueous solution | Rb | 0.11 | 0.08 | 76.7 | 5.8 | 94.1 | 0.8 |
| Example 6 | Water | Rb, Mo | 0.11 | 0.07 | 83.8 | 3.5 | 94.3 | 1.0 |
| Example 7 | Water | Rb, Ca | 0.12 | 0.07 | 78.3 | 5.7 | 95.4 | 2.1 |
| Example 8 | 0.1% NH$_3$ Aqueous solution | Rb, Mo | 0.11 | 0.13 | 81.3 | 4.6 | 95.1 | 1.8 |
| Example 9 | Water | Rb | 0.16 | 0.09 | 81.8 | 4.3 | 94.7 | 1.4 |
| Example 10 | Water | K | 0.14 | 0.19 | 81.3 | 4.5 | 94.8 | 1.5 |
| Example 11 | 0.05%NaOH Aqueous solution | Rb | 0.12 | 0.13 | 80.4 | 4.8 | 94.8 | 1.5 |
| Example 12 | 0.05% NH$_3$ Aqueous solution | Rb | 0.11 | 0.27 | 80.0 | 4.9 | 94.7 | 1.4 |
| Example 13 | 0.1% NH$_3$ Aqueous solution | Rb | 0.16 | 0.27 | 80.7 | 4.7 | 94.8 | 1.5 |
| Comparative Example 1 | Water | - | 0.13 | 0.05 | 69.5 | 6.3 | 88.4 | -4.9 |

(continued)

| | Solvent treatment and impregnation condition | | X-Ray Diffraction | | Reaction evaluation result | | | |
|---|---|---|---|---|---|---|---|---|
| | Treatment solvent | Impregnation element | P/R | Q/R | AN Yield [%] | HCN Yield [%] | AN + 3HCN | AN + 3HCN Difference from Production Example 1 |
| Comparative Example 2 | 0.05%NaOH Aqueous solution | - | 0.11 | 0.04 | 68.9 | 6.4 | 88.1 | -5.2 |
| Comparative Example 3 | 0.05% NH$_3$ Aqueous solution | - | 0.12 | 0.04 | 68.4 | 6.6 | 88.2 | -5.1 |
| Comparative Example 4 | Water | - | 0.20 | 0.19 | 73.3 | 5.4 | 89.5 | -3.8 |
| Comparative Example 5 | 0.05%NaOH Aqueous solution | - | 0.16 | 0.04 | 72.2 | 5.7 | 89.3 | -4.0 |
| Comparative Example 6 | 0.05% NH$_3$ Aqueous solution | - | 0.09 | 0.12 | 71.8 | 5.7 | 88.9 | -4.4 |
| Comparative Example 7 | 0.05%NaOH Aqueous solution | - | 0.09 | 0.27 | 77.4 | 4.6 | 91.2 | -2.1 |
| Comparative Example 8 | 0.05% NH$_3$ Aqueous solution | - | 0.13 | 0.33 | 78.9 | 4.4 | 92.1 | -1.2 |
| Comparative Example 9 | - | - | 0.18 | 0.08 | 84.2 | 3.0 | 93.2 | -0.1 |

Industrial Applicability

The catalyst of the present invention has industrial potency to be used in the production of acrylonitrile and hydrogen cyanide including a step of ammoxidating propylene.

**Claims**

1. A catalyst comprising Mo, Bi, and Fe, and satisfying, in an X-ray diffraction analysis, 0.10 < P/R < 0.18 and 0.06 < Q/R < 0.30 where P represents a peak intensity at 2θ = 22.9 ± 0.2°, Q represents a peak intensity at 2θ = 28.1 ± 0.1°, and R represents a peak intensity at 2θ = 26.6 ± 0.2°.

2. The catalyst according to claim 1, comprising a metal oxide represented by a formula (1):

$$Mo_{12}Bi_aFe_bX_cY_dZ_eO_f \qquad (1)$$

wherein X is one or more elements selected from the group consisting of nickel, cobalt, magnesium, calcium, zinc, strontium, barium, and tungsten,

Y is one or more elements selected from the group consisting of cerium, chromium, lanthanum, neodymium, yttrium, praseodymium, samarium, aluminum, boron, gallium, and indium,
Z is one or more elements selected from the group consisting of sodium, potassium, rubidium, and cesium,

a, b, c, d and e satisfy $0.1 \leq a \leq 2.0$, $0.1 \leq b \leq 4.0$, $0.1 \leq c \leq 10.0$, $0.1 \leq d \leq 3.0$, and $0.01 \leq e \leq 2.0$, respectively, and f is number of oxygen atoms required to satisfy valence requirements of other elements present.

3. The catalyst according to claim 1 or 2, further comprising a silica-containing carrier.

4. A method for producing the catalyst comprising Mo, Bi, and Fe according to any one of claims 1 to 3, comprising:

   a step of providing calcined particles obtained by calcining dried particles containing Mo, Bi, and Fe,
   a Mo removal treatment step of removing at least a part of Mo-containing components from the calcined particles,
   a step of subjecting the calcined particles from which the Mo-containing components have been removed to an impregnation operation to obtain impregnated particles, and
   a step of calcining the impregnated particles.

5. The method for producing the catalyst according to claim 4, wherein the particles are impregnated with elements by the impregnation operation, and the elements include an element dissolved out into a solvent used for the Mo removal treatment.

6. The method for producing the catalyst according to claim 4 or 5, comprising, after the step of subjecting to the impregnation operation, a step of calcining the obtained impregnated particles in the air.

7. A method for producing acrylonitrile, comprising a step of reacting propylene, molecular oxygen, and ammonia in the presence of the catalyst according to any one of claims 1 to 3.

8. The method for producing acrylonitrile according to claim 7, which is carried out in a fluidized bed reactor.

9. The method for producing acrylonitrile according to claim 7 or 8, wherein a molar ratio of ammonia and air to propylene ranges from 1.0/(0.8 to 2.5)/(7.0 to 12.0) in terms of propylene/ammonia/air ratio.

10. The method for producing acrylonitrile according to any one of claims 7 to 9, wherein the reaction is carried out within a temperature range from 300 to 550°C.

Figure 1

## INTERNATIONAL SEARCH REPORT

| International application No. |
|---|
| PCT/JP2020/013739 |

A.   CLASSIFICATION OF SUBJECT MATTER
B01J 23/887(2006.01)i; B01J 37/02(2006.01)i; B01J 37/08(2006.01)i; C07B
61/00(2006.01)i; C07C 253/26(2006.01)i; C07C 255/08(2006.01)i
FI:     B01J23/887  Z; B01J37/02  101A; B01J37/02  101Z;  B01J37/08;
        C07B61/00 300; C07C253/26; C07C255/08

According to International Patent Classification (IPC) or to both national classification and IPC

B.   FIELDS SEARCHED

Minimum documentation searched (classification system followed by classification symbols)
B01J23/887; B01J37/02; B01J37/08; C07B61/00; C07C253/26; C07C255/08

Documentation searched other than minimum documentation to the extent that such documents are included in the fields searched
    Published examined utility model applications of Japan         1922–1996
    Published unexamined utility model applications of Japan       1971–2020
    Registered utility model specifications of Japan               1996–2020
    Published registered utility model applications of Japan       1994–2020

Electronic data base consulted during the international search (name of data base and, where practicable, search terms used)

C.   DOCUMENTS CONSIDERED TO BE RELEVANT

| Category* | Citation of document, with indication, where appropriate, of the relevant passages | Relevant to claim No. |
|---|---|---|
| A | WO 2017/069119 A1 (NIPPON KAYAKU CO., LTD.) 27.04.2017 (2017-04-27) claims 1-7, paragraphs [0015]-[0022], fig. 1 | 1-10 |
| A | JP 2002-179610 A (ROHM AND HAAS COMPANY) 26.06.2002 (2002-06-26) claims 1-12, paragraphs [0021]-[0032] | 1-10 |
| A | JP 2012-245484 A (ASAHI KASEI CHEMICALS CORP.) 13.12.2012 (2012-12-13) entire text | 1-10 |

☐   Further documents are listed in the continuation of Box C.          ☒   See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 09 June 2020 (09.06.2020) | 23 June 2020 (23.06.2020) |

| Name and mailing address of the ISA/<br>    Japan Patent Office<br>    3-4-3, Kasumigaseki, Chiyoda-ku,<br>    Tokyo 100-8915, Japan | Authorized officer<br><br>Telephone No. |
|---|---|

Form PCT/ISA/210 (second sheet) (January 2015)

## INTERNATIONAL SEARCH REPORT
Information on patent family members

International application No.

PCT/JP2020/013739

| Patent Documents referred in the Report | Publication Date | Patent Family | Publication Date |
|---|---|---|---|
| WO 2017/069119 A1 | 27 Apr. 2017 | US 2019/0070591 A1 claims 1-7, paragraphs [0027]-[0035], fig. 1 EP 3366372 A1 CN 108136377 A KR 10-2018-0069052 A | |
| JP 2002-179610 A | 26 Jun. 2002 | US 2003/0187297 A1 claims 1-12, paragraphs [0037]-[0049] EP 1201636 A2 CN 1346822 A KR 10-2008-0007296 A | |
| JP 2012-245484 A | 13 Dec. 2012 | (Family: none) | |

Form PCT/ISA/210 (patent family annex) (January 2015)

**EP 3 957 394 A1**